# EUROPEAN PATENT APPLICATION

(11) **EP 2 532 704 A1**
(43) Date of publication of application: **12.12.2012**
(21) Application number: 11168933.7
(22) Date of filing: 07.06.2011
(51) Int. Cl.: C08J 7/04, C12N 11/14, C09D 183/04, C09D 183/08, C09D 4/00

(54) **Coated polymer system and methods for its production**

(71) Applicant: Fachhochschule Bielefeld, 33615 Bielefeld (DE); Technische Universität, 09599 Freiberg (DE)
(72) Inventor: Kroke, Edwin, 09633 Halsbrücke (DE); Patel, Anant, 33604 Bielefeld (DE); Wiltzsch, Conny, 09618 Brand-Erbisdorf (DE)
(74) Representative: Kröncke, Rolf

(57) **Abstract**

The present invention relates in a first aspect to a coated polymer system comprising silica-coated polymer and functional components. Said coating of the polymer is a silica-coating obtained by using Si-N precursor having at least two amino groups and, in addition, coating is affected in the presence of the functional component. In another aspect, the present invention relates to a method for preparing the coated polymer systems containing functional components comprising the step of coating the polymer with a Si-N precursor having at least two amino groups in the presence of the functional component.

## Description

The present invention relates in a first aspect to a coated polymer system comprising silica-coated polymer and functional components. Said coating of the polymer is a silica-coating obtained by using Si-N precursor having at least two amino groups and, in addition, coating is affected in the presence of the functional component. In another aspect, the present invention relates to a method for preparing the coated polymer systems containing functional components comprising the step of coating the polymer with a Si-N precursor having at least two amino groups in the presence of the functional component.

### Prior art

Immobilisation of biomolecules and cells within polymer matrices has gained considerable importance in various fields of biotechnical processes. For example, immobilised biomolecules present e.g. in biopolymers are relevant in the field of red, green and white biotechnology. Beside the use of encapsulated biologicals including catalytic antibodies, DNA, RNA, antigens, life bacterial, fungal, plant and animal cells as well as whole protozoa for various uses in red and green biotechnology, bioencapsulation is important in the field of white biotechnology. For example, said encapsulated active components include catalytically active components, like catalysts or enzymes useful in all types of reactions. In addition, bioencapsulation may ensure sustained release as well as improved and extended activity of the encapsulated functional components.

Moreover, other applications are considered in the field of optical and electrochemical field, in diagnostic devices but also in the green biotechnology.

Encapsulation of yeast is described for various purposes, e.g. in EP 173915 a biocatalyst having immobilised cells as well as a process for preparing the same is disclosed. Said process comprises the formation of a core containing the cells surrounded by a gel forming a protective layer free of the cells while no permeability to the cells is given and the protective layer enclose fully the core containing the cells. Various other sol-gel biopolymer processes are described. In Coradin T., et.al., Applied Microbiology and Biotechnology, 2003, June, 61 (5-6), 429-34 a review of silica-alginate composites for microencapsulation is provided. As explained therein, one of the major challenges of microencapsulation techniques is to immobilise living cells retaining their activity behind a semi-permeable membrane thereby allowing their use in the various applications identified above. Numerous biopolymers as well as synthetic polymers are described in the art useful for encapsulation of functional components, in particular, living cells or bioactive materials.

Alginate encapsulation was reported for more than 30 years. Strengthening of alginate microcapsules with silica was reported almost 10 years ago. Coating with silica allows to associate soft biocompatible components, like alginate with a tough, thermostable non-swelling component, like silica. In addition, it is identified by Coradin et.al. that combination of silica coating with biopolymers, like alginate, allows to improve capsule stability while controlling membrane diffusion properties as well as allowing efficient cell immobilisation and transplantation.

However, sol-gel encapsulation of living material is still a challenging matter. As reviewed by Livage et.al., Reviews in Mineralogy & Geochemistry, 2006, Vol 64, 315-332, encapsulation of living cells, e.g. in oxide glasses, is handled by various problems arriving from the type of silica precursor and depending on the processes for preparing the sol-gel capsules coated with silica. A comparison of different approaches for sol-gel encapsulation of bacteria is provided by Coiffier et.al., J. Mater. Chem., 2001, 11, 2039-2044. It is concluded that aqueous silica matrices appear to be less detrimental to cell viability compared to the usual alkoxide route. However, it is identified that the amount of methanol produced by hydrolysis and condensation of TMOS (the Si precursor) could exceed the tolerated threshold, thus, being detrimental for the functional components encapsulated in the polymer system.

In addition, Coradin et.al., Current Nanoscience, 2006, 2, 219-230 discuss various problems when preparing sol-gel biopolymer silica composites. It is identified that obstacles in the preparation processes are the presence of alcohol in the reaction media, unfavourable pH, temperature, etc. As identified, various approaches were proposed including thin film deposition, evaporation of alcohol before biological addition, synthesis of new precursors bearing biocompatible alcohol groups as well as substitution of silicon alkoxide by aqueous precursors, like silicate or colloidal silica. However, the outcome of said various approaches are still not favourable. That is, the use of TMOS as a typical precursor requires conditions for hydrolysis and condensation including temperatures of above 100°C and reaction conditions including solubilisation in solvents, typically organic solvents, having a pH value in the acidic range, in particular, at pH 2 or below. Furthermore, organic solvents, like alcohols such as ethanol or methanol, ethers such as THF or other organic solvents are required for solubilisation of TMOS and other Si-O based precursor molecules.

For example, in WO2011/011468 a method for encapsulating cells is described using TMOS as Si-O based precursor trying to circumvent the drawbacks of TMOS. That is, the method disclosed therein requires the use of a silica-saturated buffered media. In addition, the method requires that after hydrolysis of the precursor molecules, the by-products, in particular, the alcohol formed in the solution, like methanol in case of TMOS, is removed by evaporation before contacting the saturated solution with the cell material in order to avoid any negative influence of said by-products on the cell material.

Recently, Meunier et.al., Chem. Commun., 2010, 46, 3843-3859, discusses the use of living hybrid materials capable of energy conversion and CO₂ assimilation. As identified therein, there is still a problem on controlling the formation of gels from aqueous silica precursor and silica nanoparticles for entrapping the living or functional components. It is identified further that there is still an ongoing need for providing polymer systems, preferably coated polymer systems comprising a silica coating containing functional components, preferably in form of capsules having improved activity showing enhanced lifetime.

The present invention is directed to new coated polymeric systems containing functional components, in particular, pH or temperature sensitive functional components whereby said polymer is coated with a silica coating.

In another aspect, the present invention is directed to methods for preparing coated polymeric systems containing functional components whereby coating of the polymeric system with silica is conducted in the presence of the functional component.

### Brief summary of the present invention

In a first aspect, a coated polymer system is provided. The coated polymer system according to the present invention comprises silica-coated polymer and functional components. Preferably, the functional components are encapsulated fully in the polymer and/or the silica-coating.

It is preferred that the polymer is a biopolymer, in particular, a biopolymer selected from alginates, carrageenans or other polysaccharides, proteins or mixtures thereof.

In addition, the functional component is preferably a biological component, in particular, whole cells or cellular components, e.g. enzymes.

The present inventors recognised that it is possible to obtain improved coated polymer systems, preferably, capsules or particles, where the silica coating of said polymer system is obtained by polymerisation of Si-N precursors having at least two amino groups. Said Si-N precursors are favourable in view of the production process, like hydrolysis and condensation of the Si-N precursor. In particular, the Si-N precursors are favourable in view of the sensitivity of functional components, in particular, biological components, like whole cells or enzymes, to low pH values, acids as well as high temperature and compounds being harmful or toxic to the functional components, like the by-products methanol or ethanol formed during the hydrolysis of TMOS.

In another aspect, the present invention relates to a method for the preparation of a coated polymer system with functional components comprising the step of coating the polymer with Si-N precursor having at least two amino groups in the presence of said functional components. In contrast to processes described in the art using Si-O precursor, the Si-N precursor allows formation of the coating at reaction conditions favourable for the functional components present in the polymer system. In particular, it is possible to conduct the process at starting reaction conditions like neutral pH, temperatures below 50°C, etc, without the production of harmful or toxic by-products or laborious steps to remove the same. Hence, the reaction conditions for forming the silica coating is less detrimental to the functional components compared to the use of Si-O precursor only. In addition, the method is efficient, cost effective and simple.

### Detailed description of the present invention

In a first aspect, a coated polymer system comprising silica-coated polymer and functional components is provided. Said coated polymer system is characterised in that the coating of the polymer is a silica coating whereby said silica coating is obtained by using a Si-N precursor having at least two amino groups and whereby said coating is conducted in the presence of the functional components. In this connection, the term "polymer system" as used herein, refers to a polymer system of known and suitable polymers forming e.g. a sol-gel polymer whereby said term does not include silica-based polymers or other inorganic polymers. Further, the term "silica coating" or "silica-coated" refers to a coating or coat of silica based polymers.

The present inventors recognised that the use of Si-N precursor instead of known Si-alkoxide precursor, like tetramethoxysilane (TMOS) or tetraethoxysilane (TEOS) allows to provide new silica-coated polymer systems comprising said silica-coated polymer and functional elements. The coated polymer system according to the present invention demonstrates improved properties with respect to functionality and activity of the functional components present in said coated polymer system. For example, in case of living cells, the vitality of said cells present in said silica-coated polymer system based on Si-N precursor is improved compared to the silica-coated polymer system based on Si-O precursor.

The silica coating of the polymer is obtained by hydrolysis and subsequent condensation of the Si-N precursor molecules forming a silica coating.

The present inventors recognised that substituting at least a part of known Si-O precursor with Si-N precursor having at least two amino groups result in coated polymer systems having a silica coating wherein the environmental and reaction conditions are less detrimental to the functional components than using Si-O precursor molecules.

As used herein, the term "Si-O precursor" refers to silane derivatives containing at least two reactive -OR residues with R being H or any other substituent, like hydrocarbon, as a leaving group during polymerisation.

In addition, the term "Si-N precursor" refers to silane derivatives having at least two amino groups whereby said amino groups may be primary, secondary or tertiary amino groups. The Si-N precursor useful according to the present invention has at least two amino groups allowing condensation of said precursor molecules to silica polymers whereby primary amines, secondary amines or ammonia are generated.

The coated polymer system is preferably a coated polymer system wherein the content of nitrogen in the coated polymer system is at least 5%, like at least 10% higher than the content of nitrogen in the coated polymer system being coated with a Si-O precursor. That is, it is preferred that the content of nitrogen in the coated polymer system comprising silica-coated polymer and functional components is at least 5%, like at least 10% higher than the content of nitrogen in the coated polymer system comprising silica-coated polymer and functional components wherein said coating is obtained by condensation of Si-O precursor. Preferably, the content of nitrogen is at least 15% higher, like at least 20% higher, in particular, at least 25% higher. Alternatively or in addition, the amount of nitrogen in the coated polymer system in the absence of a functional component is at least 0.1 wt.-%, like at least 0.2, 0.3 or at least 0.5 wt.-% of the weight of the total coated polymer system not containing functional components. It is preferred that the amount of nitrogen is at least 1.0 wt.-% of the weight of the total coated polymer system, like 1.5 wt.-% or at least 2.0 wt.-%.

In another preferred embodiment, the coated polymer system according to the present invention is in form of capsules, particles, films or fibres. For example, it is preferred that the coated polymer system is a sol-gel coated polymer system, like silica-coated sol-gel capsules or particles.

In this respect, the term "capsules" as used herein in the following includes capsules, particles, films, fibres, beads, hollow beads, multi-layer systems or blocks unless otherwise indicated.

Further, the term "coated polymer system" as used herein refers to a polymer system whereby the silica coating may enclose fully the core formed by the polymer or may be present on one side of the polymer system only depending on the form of the polymer system. That is, in case of capsules, the silica coating is present on the surface of said capsules covering preferably fully said capsules.

In another embodiment, the polymer system may be a system coated on one side only, for example when being in form of a matrix, membrane or sheet whereby the coating is on one side of said matrix, membrane or sheet while the functional components may be present on the other side and/or same side of said sheet or membrane, respectively, or may be present in the polymer and/or the silica coating.

It is preferred that the functional components are encapsulated fully by the polymer and/or the silica-coating.

The polymer present in the coated polymer system is preferably a biopolymer. The polymer may be a sol-gel polymer as known in the art. It is particular preferred that the polymer is a biopolymer selected from alginate, carrageenate or other polysaccharides or proteins or mixtures thereof.

As noted before, it is particularly preferred that the polymer is a sol-gel polymer. The polymer system may be present as a hollow polymer system, for example, hollow capsules, like hollow spheres. Alternatively, the polymer system may be a gel matrix or a bulk body. The skilled person is well aware of the different forms and embodiments of polymer systems.

The functional component present in the coated polymer system according to the present invention may be present in the core of the hollow capsule or present in the core area of the gel or the bulk body. Alternatively or in addition, the functional components may be present in the outer areas of the capsule including the silica coating.

That is, according to another embodiment of the present invention, the coated polymer system according to the present invention is a system whereby the functional component, in particular, a biological active material, is present in the silica coating formed from the Si-N precursor having at least two amino groups. Alternatively or in addition, the coated polymer system is a polymer system whereby said polymer is a sol-gel biopolymer containing said functional component and whereby said sol-gel polymer containing said functional component is coated with a silica coating from the Si-N precursors having at least two amino groups.

In this connection, the term "functional component" refers to whole cells and cell compartments, proteins, in particular, enzymes, nucleic acids, secondary metabolites. In particular, the functional component is a component, e.g. molecule, which is sensitive to low pH values and/or high temperature, e.g. above 50°C, like above 40°C as well as being sensitive to harmful or toxic by-products like methanol or ethanol. That is, the functionality, e.g. the activity or specific characteristic, of said functional component is pH-sensitive and/or temperature-sensitive as well as being sensitive to toxic or harmful compounds. Thus, the desired properties (specific characteristic) of said functional components may be impaired or even destroyed at pH or temperature conditions required for hydrolysis and condensation of Si-O precursor molecules. In a preferred embodiment, the functional components are biological components, in particular, cells, cellular components, and/or biologically active molecules, in particular, enzymes.

For example, when the coated polymer system according to the present invention is intended for use in white biotechnology, e.g. as a catalyst or as part of cleaning agents, the functional component is an enzyme or other biocatalyst or similar component or even a living cell. Alternatively, in case the functional component is a biocatalyst, said biocatalyst may be useful in fermentation, e.g. fermentation of food. In another embodiment of the present invention, the functional component is a biosensor, a cell or other types of living organisms, in particular, cells or viruses encoding, containing and/or producing active components or cause/trigger a desired effect like change of the pH, electric field, luminescence, colour, etc. Further, the functional component may display toxicity against fungi, bacteria and/or insects. In addition, the functional component may be enzymes useful in detergents or fertilizer etc.

In another embodiment, the functional component of the present invention may be on one side of the polymer system while the coating is on a different side of said system. In an alternative embodiment, the functional component is present in the polymer or the silica coating.

In another embodiment of the present invention, the functional component may be a harmful or a toxic component, e.g. a pesticide, like fungicide or bactericide, insecticides or sporocides. In another embodiment of the present invention, the functional component may be a fertilizer, a detergent, an odorous substance, essence (gustatory substance), a colorant, a luminescent substance, a lubricant, a pharmaceutically active substance or a cosmetically applicable substance.

Furthermore, the polymer system according to the present invention may be a system having a smart material effect. This means that the polymer system itself, e.g. in form of a gel, may react with the environment, thus a release of the functional component may be triggered by temperature changes, mechanical activation (isotropic or anisotropic pressure, shear, friction, torsion), light or other radiation (long wavelength, electromagnetic radiation, like IR, microwave, or short wavelength radiation like UV-light or X-ray, or particulate beams like alpha or beta radiation, ion beams), magnetic or electric fields or chemical substances.

It is preferred that the functional component in the coated polymer system according to the present invention has a remaining functionality after coating of at least 50%, like at least 60%, e.g. at least 70%, like at least 80%, in particular, of at least 90%, like 95% compared to the activity of the functional component before incorporation into the coated polymer system.

In this connection, the term "activity" or "functionality" refers to a specific characteristic of the functional component. Said characteristic may be a biological activity, like an enzymatic activity or other biocatalytical activity. Alternatively, said activity may be a biosensor, a fertilizer, a detergent, an odorous substance, essence (gustatory substance), a colorant, a luminescent substance, a lubricant, a pharmaceutically active substance or a cosmetical, an producer and/or emitter of an active substance, providing toxicity against fungi, bacteria, insects etc.

The present inventors identified that using the Si-N precursor molecules instead of Si-O precursor molecules allow to obtain coated polymer systems comprising silica-coated polymers and functional component under reaction conditions more favourable for said functional components present in the coated polymer system according to the present invention. In contrast to the required reaction conditions when using silane components, i.e. reaction conditions at or below pH 2 and temperatures above 100°C, the hydrolysis and condensation of the Si-N precursor molecules, like aminosilane molecules,can be performed quickly at room temperature and can be started at neutral pH values, thus, allowing to retain the activity of the functional component. In addition, the toxicity of by-products of hydrolysis and condensation of the Si-O precursor, e.g. methanol or ethanol, is avoided. In contrast, the side products of the hydrolysis and condensation of the Si-N precursor, like secondary amines, primary amines or ammonia are less harmful or toxic to the functional components present in the coated polymer system.

The Si-N precursors are preferably aminosilane components or oligo- and polysilazane components or mixtures thereof.

That is, the Si-N precursor is preferably an aminosilane component of the general formula (I)

(I) AₓSi(NR₁R₂)₄₋ₓ

wherein A is independently selected from hydrogen, unsubstituted or substituted hydrocarbon, optionally containing heteroatoms, in particular, O, N, S, Si, Cl, Br, F and/or P; said hydrocarbon may be a saturated or unsaturated hydrocarbon in form of a linear, branched or cyclic hydrocarbon residue, furthermore, A may be an aromatic residue, preferably A is independently selected from optionally substituted C₁-C₂₀-alkyl-, C₆-C₁₂-aryl, in particular preferred a methyl, ethyl, propyl, butyl or phenyl residue. Substituents include hydrocarbons, like branched or linear alkyl, alkenyl alkoxy, hydroxyl, halogens, but also amine, amid, imid groups as well isocyanate groups, etc. Preferably, the hydrocarbon is a hydrocarbon of C₁ to C₆ including C₂, C₃, C₄ and C₅.
x is an integer of 0, 1 or 2

R₁ and R₂ are independently from each other hydrogen, unsubstituted or substituted hydrocarbon, optionally containing heteroatoms, in particular, O, N, S, Si, Cl, Br, F and/or P; said hydrocarbon may be a saturated or unsaturated hydrocarbon in form of a linear, branched or cyclic hydrocarbon residue, furthermore, R₁ and R₂ may be an aromatic residue, preferably R₁ and R₂ is independently selected from hydrogen,optionally substituted C₁-C₂₀-alkyl-, C₆-C₁₂-aryl, in particular preferred a hydrogen, methyl, ethyl, propyl, butyl, hexyl or phenyl residue, whereby the substituents are preferably the substituents as defined above namely hydrocarbons, like branched or linear alkyl, alkenyl alkoxy, hydroxyl, halogens, but also amine, amid, imid or ether groups as well isocyanate groups, etc. further including functionalized primary amines.

In particular, it is preferred that A is methyl, ethyl or propyl and/or R₁ and R₂ are independently from each other selected from hydrogen, n-propyl, iso-propyl and hexyl whereby at least one of R₁ and R₂ are not hydrogen.

It is preferred that x is an integer of 0 or 1, thus the aminosilane is a triaminosilane or tetraaminosilane of A-Si(NR₁R₂)₃ or Si(NR₁R₂)₄.

In another aspect, the Si-N precursor is an oligo- and/or polysilazane. For example, the oligo- or polysilazane is an perhydrated oligo- or polysilazane based on (SiH₂-NH₂)ₙ chains or rings. Oligo- und polysilazanes are inorganic polymers consisting of Si-N-Si-chains. Unsubstituted, i.e. perhydrated oligo- und polysilazanes based on (SiH₂-NH₂)ₙ chains and rings are very reactive and, thus, are preferred according to the present invention.

For example, perhydropolysilazane (PHPS) as given below are used:

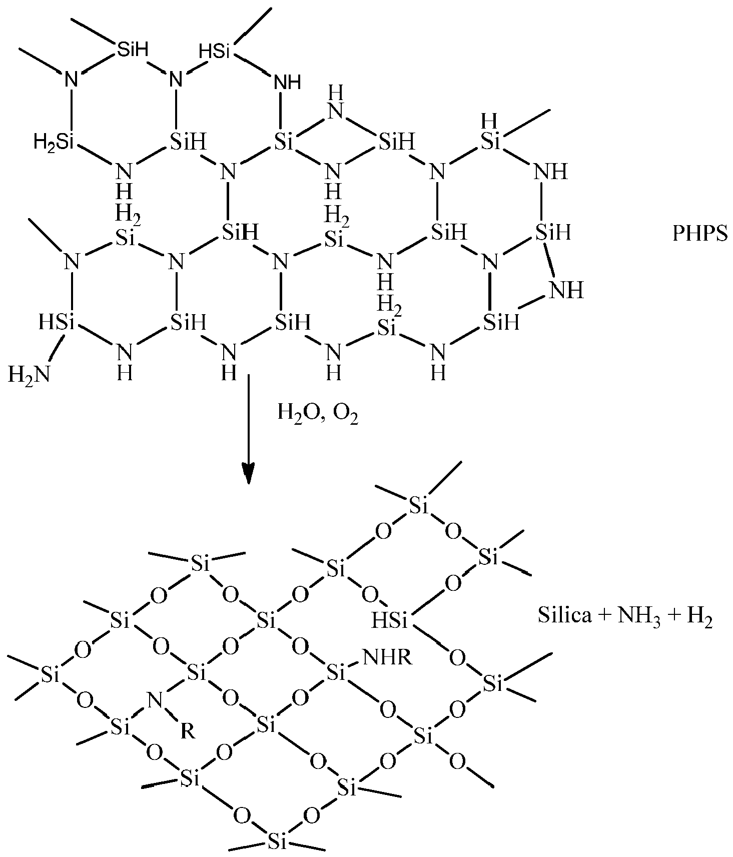

It is preferred that PHPS of the general formula (II) are used.

-(SiR¹R²-NR³)ₙ- (II)

wherein R¹, R², R³ are identical or different from each other and are independently from each other seleceted from hydrogen, unsubstituted or substituted hydrocarbon, optionally containing heteroatoms, in particular, O, N, S, Si, Cl, Br, F and/or P; said hydrocarbon may be a saturated or unsaturated hydrocarbon in form of a linear, branched or cyclic hydrocarbon residue, furthermore, R₁ and R₂ may be an aromatic residue, preferably R₁ and R₂ is independently selected from optionally substituted C₁-C₂₀-alkyl-, C₆-C₂₄-aryl, in particular preferred a methyl, ethyl, propyl, butyl, hexyl or phenyl residue, whereby the substituent may be any one as defined above for R (R1, ...). In addition, R¹, R², R³ may represent an aprotic group, like halogens, -CN, N02, OR, COOR, heterocyclic groups, or alkyl, alkenyl, or alkinyl groups, whereby R is a linear or branched, saturated or unsaturated hydrocarbon, like C1-C6 alkyl, C1 to C₂₀ alkenyl.

Preferred components of the aminosilane group are -NHR with R = alkyl, especially R = methyl, ethyl, n-propyl, n-butyl, iso-butyl, t-butyl, n-pentyl, n-hexyl, cyclohexyl, phenyl, para-methylphenyl.

Further, the Si precursor molecules may comprise bridging components like halogenated silanes as well known to the skilled person.

The Si-N precursor molecule release primary amine, secondary amines or ammonia depending on the residues present at the N-atom of the amino group, which are less detrimental to the functional components than the side products released when using Si-O precursor molecules, like methanol or ethanol. Moreover, due to higher reactivity of the aminosilanes, the coating process is much faster under the same pH- and temperature conditions.

The primary or secondary amine hydrocarbon components or ammonia released as side products of hydrolysis and condensation of Si-N precursors compounds are less reactive and less harmful or toxic to the functional component. In addition, hydrolysis and condensation of the Si-N precursor is possible at lower temperature and at pH values of neutral pH. Moreover, the coating processes are fast and efficient, thus, preserving functional activity of the functional components present in the system according to the present invention. The coated polymer systems according to the present invention are permeable for molecules depending on the grade of condensation of the silica coating and the polymer. In addition, additives may be present regulating thickness and permeability as well. That is, it is possible by selecting appropriate reaction conditions for polymerisation and suitable Si-N precursor molecules to obtain coated polymer systems having a specific cut off range of permeability. For example, the coating may be permeable for nutrients for the living cells or the reactants or educts of the biocatalysts present in the coated polymer system. For example, when using living cells or cellular components, e.g. enzymes, the coating and/or the polymer itself is permeable for the reactant and the products while the biocatalyst or the cells or cellular components remain in the polymer system. Thus, the present invention provides coated polymer systems, e.g. in form of capsules or in form of matrices, membranes or sheets having a predetermined permeability, for example maybe semi-permeable for components like the reaction products, while the functional components present in the coated polymer systems remain in said system. Such systems are particularly useful when using biocatalysts or biosensors as a functional component.

In another aspect, the present invention provides a method for the preparation of a coated polymer system with functional components comprising the step of coating the polymer with Si-N precursor having at least two amino groups in the presence of said functional components. The functional component may be present in the polymer or may be separated or encapsulated by the polymer while condensation with Si-N precursor for forming a silica-based coating on the polymer occurs. In addition or alternatively, the functional components may be present in the coating.

It is preferred that the Si-N precursor is an aminosilane or an oligo- or polysilazane as described herein.

In a preferred embodiment, the method is a method wherein the polymer system to be coated is a sol-gel biopolymer and the coating of the biopolymer is performed after formation of the sol-gel biopolymer whereby the functional component is present in the biopolymer and/or in the coating.

In another preferred embodiment of the present invention, the components forming the polymer, the Si-N precursor having at least two amino groups and the functional components are mixed before forming the polymer and/or the coating. Thus, the coated polymer system according to the present invention may contain silica structures throughout the polymer system.

It is preferred that the coating is performed at a pH of 2 to 12, preferably 4 to 10. It is particular preferred that the pH value is in the range of 6 to 8 when starting hydrolysis and condensation the Si-N precursor molecules. Thus, it is possible to incorporate pH-sensitive functional components into the coated polymer system according to the present invention. Further, it is preferred that the temperature is in a range of from -20°C to 80°C, in particular, 0°C to 60°C. It is particular preferred that the temperature for effecting hydrolysis and condensation of the Si-N precursor is in the range of 20°C to 40°C. Thus, it is possible to incorporate temperature-sensitive functional components, e.g. biological components like cells, cellular components or other biologically active molecules, e.g. enzymes or other biocatalysts, into the coated polymer system according to the present invention.

Furthermore, in another preferred embodiment, the functional component, in particular a biological active component, is present in the silica coating formed by the Si-N precursor having at least two amino groups.

In a preferred embodiment, the coating is affected in a fluidised bed process. Of course, other techniques known for the preparation of coated polymer systems may be applied. In particular, methods known for the preparation of suitable gel-sol polymers, in particular, biopolymers are well known in the art and may be used for the present invention. The coated polymer system according to the present invention is in a particular preferred embodiment a hydrogel. The coated polymer systems according to the present invention having a silica-based coating obtained by using Si-N precursor allow to provide coated polymer systems having a good thermal and mechanical stability. In addition, it has been recognised that these coated polymer systems according to the present invention allow to incorporate living cell material remaining substantially active after incorporation.

The invention will be described further by examples without limiting the present invention.

### Examples

### Example 1

Alginate capsules were prepared according to state of the art: Briefly, a 2 (w/w) % sodium alginate solution was dripped with a syringe into a stirred solution of 2 (w/w) % CaCl₂ and left to crosslink for 20 min. The resulting beads were sieved from the crosslinker solution.

### Example 2 Coating of alginate capsules with a solution of 1 % tetra(n-propylamino)silane:

a) preparation of aminosilanes Si(NHₙPR₄) according to C. Wiltzsch et al., Dalton Trans. 2009, 5474-5477.

   SiCl₄ + 8 NH₂CH₂CH₂CH₃ → Si(NHCH₂CH₂CH₃)₄ + NH₂CH₂CH₂CH₃*HCl
b) coating of alginate capsules

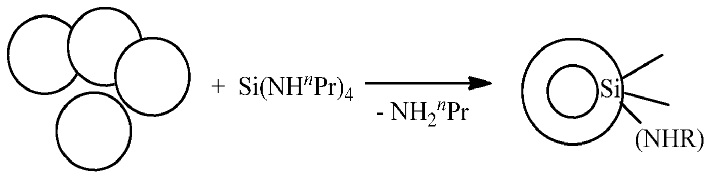

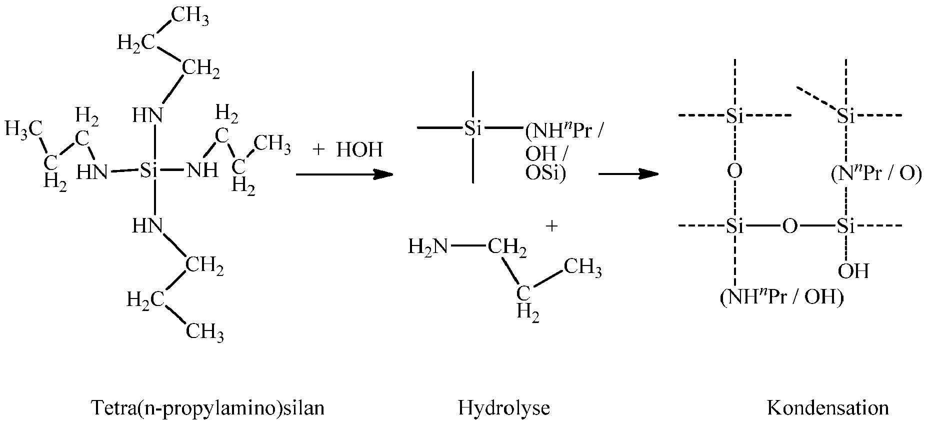

In a Schlenk-filtration apparatus 25 alginate capsules containing 2 % (w/w) yeast cells were placed on a glass drip followed by adding a 1 % tetra(n-propylamino)silane solution with hexane as solvent. After one minute an overload pressure with argon was applied to remove the excess reaction solution from the capsules. After washing two times with hexane the capsules were stored in sterile sample glasses.

The determination of viability of the yeast cells after coating of the capsules was done by growing cells on Standard I nutrient agar. For effecting growing of the cells, the capsules were placed on the culture medium. For a survey of the permeability of the coating with respect to the yeast cells present in the capsules, the capsules were divided in halves and placed with the cut face on the culture medium. Growing was effected for 15 hours at 28°C in an incubator. Vitality of the yeast cells were determined by macroscopically based on the formation of yeast colonies. To proof that the colonies are yeast colonies, cells were taken from the grown colonies and were examined under the microscope. The proof for vitality of the yeast was done by staining with Serva-blue.

It was determined that 100 % of the encapsulated yeast cells were able to grow after coating with a 1 % tetra(n-propylamio)silane solution. It was also shown that the same results were obtained from whole or cut capsules.

### Example 3 Coating of alginate capsules with a solution of 5 % tetra(n-propylamino)silane:

In a Schlenk-filtration apparatus 25 alginate capsules containing 2 % (w/w) yeast cells were placed on a glass drip followed by adding a 1 % tetra(n-propylamino)silane solution with hexane as swept. After one minute an overload pressure with argon was applied to remove the excess reaction solution from the capsules. After washing two times with hexane the capsules were stored in sterile sample glasses.

The determination of viability of the yeast cells after coating of the capsules was done as described in example 2.

It was determined that nearly 100 % of the encapsulated yeast cells were able to grow after coating with a 5 % tetra(n-propylamio)silane solution.

### Example 4 Coating of alginate capsules with a solution of 10 % tetra(n-propylamino)silane:

In a Schlenk-filtration apparatus 25 alginate capsules containing 2 % (w/w) yeast cells were placed on a glass drip followed by adding a 10 % tetra(n-propylamino)silane solution with hexane as swept. After one minute an overload pressure with argon was applied to remove the excess reaction solution from the capsules. After washing two times with hexane the capsules were stored in sterile sample glasses.

The determination of viability of the yeast cells after coating of the capsules was done as described in example 2.

It was determined that nearly 100 % of the encapsulated yeast cells were able to grow after coating with a 10 % tetra(n-propylamino)silane solution.

The amount of silicon per capsule was determined. Silicon analyses were carried out according to the following procedure:
The organic matrix was destroyed under pressure and a temperature of 200°C with nitric acid. The formed silicon dioxide was dissolved in sodium hydroxide at 150°C. Detection of silicon was realized via ICP-AES (inductively coupled plasma-Atomemissionsspektroskopie).

| Probe | coated capsule |
|---|---|
| µm Si/capsule | 0.60 |

### Example 5 Coating of alginate capsules with a 100 % tetra(n-propylamino)silane solution.

In a Schlenk-filtration apparatus 25 alginate capsules containing yeast cells were placed on the glass drip. Thereafter, pure tetra(n-propylamino)silane was added. After a minute, the reaction solution was removed from the capsules by filtration using argon with overload pressure. After washing two times with hexane, the capsules were removed and stored in sterile sample glasses.

The determination of the viability of the yeast cells were done as described in example 2.

### Example 6 Coating of alginate capsules with a solution of 10 % tetra(n-hexylamino)silane solution

a) preparation of the amino silane Si(NHₙHex)₄. (analogous to C. Wiltzsch et al., Dalton Trans. 2009, 5474-5477.)

   SiCl₄ + 8 NH₂(CH₂)₅CH₃ → Si[NH(CH₂)_{S}CH₃]₄ + NH₂(CH₂)₅CH₃*HCl
b) coating of the alginate capsules

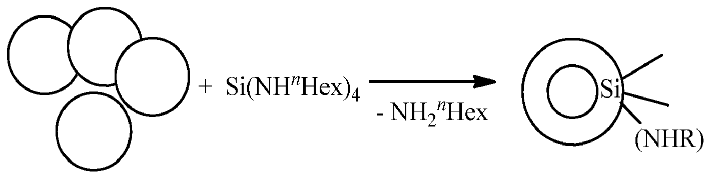

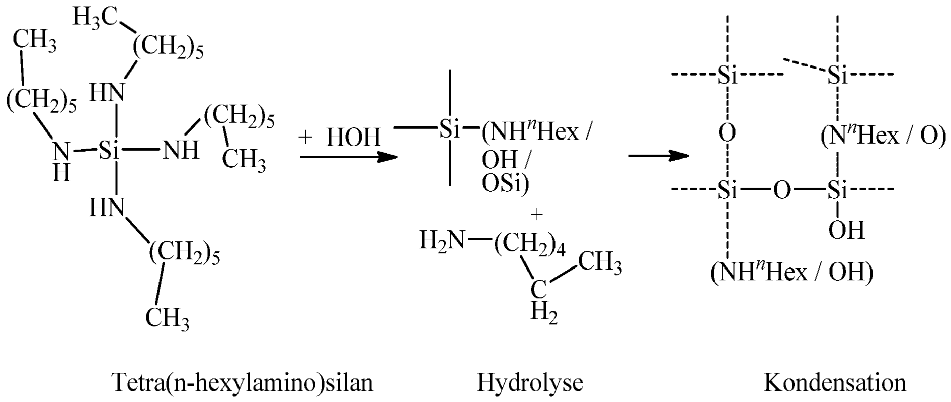

In a Schlenk-filtration apparatus 25 alginate capsules containing 2 % (w/w) yeast cells were placed on a glass drip followed by adding a 10 % tetra(n-hexylamino)silane solution with hexane as solvent. After one minute an overload pressure with argon was applied to remove the excess reaction solution from the capsules. After washing two times with hexane the capsules were stored in sterile sample glasses.

The determination of viability of the yeast cells after coating of the capsules was done as described in example 2.

### Example 7 Coating of alginate capsules with 100 % tetra(n-hexylamino)silane.

In a Schlenk-filtration apparatus 25 alginate capsules containing 2 % (w/w) yeast cells were placed on a glass drip followed by adding pure tetra(n-hexylamino)silane solution with hexane as swept. After one minute an overload pressure with argon was applied to remove the excess reaction solution from the capsules. After washing two times with hexane the capsules were stored in sterile sample glasses. Coating was visible by naked eyes.

The determination of viability of the yeast cells after coating of the capsules was done as described in example 2.

### Example 8 Coating of alginate capsules with a mixture of tetra(n-propylamino)silane and tri(n-propylamino)methylsilane (1:1).

In a Schlenk-filtration apparatus 25 alginate capsules containing 2 % (w/w) yeast cells were placed on a glass drip followed by adding the mixture of tetra(n-propylamino)silane and tri(n-propylamino)methylsilane with hexane as solvent. After one minute an overload pressure with argon was applied to remove the excess reaction solution from the capsules. After washing two times with hexane the capsules were stored in sterile sample glasses.

The determination of viability of the yeast cells after coating of the capsules was done as described in example 2.

### Example 9 Si-based polymer coating of alginate capsules with a 1 % solution of 20 % PHPS in xylol.

In a Schlenk-filtration apparatus 10 alginate capsules containing 2 % (w/w) yeast cells were placed on a glass drip. Thereafter, 1 ml 20 % PHPS in xylol being diluted with hexane to 10 ml, were added. After a minute the reaction solution was removed from the capsules by filtration with the help of an overload pressure of argon. After washing two times with hexane, the capsules were removed and stored in sterile sample glasses.

The determination of viability of the yeast cells after coating of the capsules with a done by cultivation of cells on Standard I nutrient agar. The capsules were placed on the culture medium. For determining permeability of the coating with respect to the yeast cells present in the capsules, the capsules were halved and were placed with the cut face on the culture medium. For cultivation, the samples were incubated for 15 hours at 28°C in an incubator. Viability of the yeast cells was determined macroscopically by determining the formation of yeast colonies. The proof that the colonies are yeast cells; cells were picked from the grown colonies and examined under the microscope.

It was determined on a qualitative basis that approximately 100 % of the yeast cells present in the capsules grows on the culture medium. This was independent from the fact whether whole or cut capsules were examined.

### Example 10 Si-based polymer coating of alginate capsules with a mixture of (0.5 % mixture of 20 % PHPS in xylol) and 0.5 % tetra(n-propylaminosilane) solution.

In a Schlenk-filtration apparatus 10 alginate capsules containing 2 % (w/w) yeast cells were placed on a glass drip. Thereafter, 1 ml 20 % PHPS in xylol being diluted with hexane to 10 ml, were edit. After a minute the reaction solution was removed from the capsules by filtration with the help of an over pressure of argon. After washing two times with hexane, the capsules were removed and stored in sterile sample glasses.

The determination of viability of the yeast cells after coating of the capsules with a done by cultivation of cells on Standard I nutrient agar. The capsules were placed on the culture medium. For determining permeability of the coating with respect to the yeast cells present in the capsules, the capsules were halved and were placed with the cut face on the culture medium. For cultivation, the samples were incubated for 15 hours at 28°C in an incubator. Viability of the yeast cells was determined macroscopically by determining the formation of yeast colonies. The proof that the colonies are yeast cells; cells were picked from the grown colonies and examined under the microscope.

It was determined on a qualitative basis that approximately 100 % of the yeast cells present in the capsules grows on the culture medium. This was independent from the fact whether whole or cut capsules were examined.

### Example 11 Coating of alginate capsules using different concentrations of tetra(n-propylamino)silane solution.

In a Schlenk-filtration apparatus 25 alginate capsules containing yeast cells were placed on a glass drip. Thereafter, a tetra(n-propylamino)silane solution in a concentration as indicated in table 2 below or TEOS solved in hexane as solvent, were added. After a predetermined time as indicated in the table below the reaction solution was removed from the capsules by filtration using argon under overload expression. After washing two times with hexane and THF, the capsules were placed in sterile sample glasses.

**Table 2**

| **sample** | **treatment** | **content N (%)** | **content C (%)** | **content H (%)** |
|---|---|---|---|---|
| 1 | non | 5.1 | 32.9 | 6.18 |
| 2 | 10 % SiN₄ 1 minute | 5.4 | 34.8 | 6.31 |
| 3 | 10 % SiN₄ 30 minutes | 6.0 | 39.3 | 6.74 |
| 4 | 100 % SiN₄ 30 minutes | 8.3 | 40.7 | 7.3 |
| 5 | 10 % TEOS 1 minute | 5.3 | 35.4 | 6.56 |
| 6 | 10 % TEOS 30 minutes | 5.1 | 31.95 | 6.99 |
| 7 | 100 % TEOS 30 minutes | 5.3 | 38.3 | 6.77 |

### Example 12 Coating of alginate capsules with different concentrations of tetra(n-propylamino)silane solution.

In a Schlenk-filtration apparatus 25 alginate capsules not containing any yeast cells, were placed on a glass drip. Thereafter, tetra(n-propylamino)silane solution in hexane as solvent with the concentration as indicated in the table, was added. After the time indicated in the table 3, the reaction solution was removed from the capsules by filtration using argon under over expresser. After washing two times with hexane and THF, the capsules were stored in sterile sample glasses.

The content of elemental C, H and N in the samples was determined the system vario Micro cube (Fa. Elementar, Hanau, Germany). The amount of elemental C, H and N are given in table 3.

**Table 3**

| **sample** | **treatment** | **content N (%)** | **content C (%)** | **content H (%)** |
|---|---|---|---|---|
| 1 | non | 0.03 | 20.4 | 4.32 |
| 2 | 10 % SiN₄ 1 minute | 0.45 | 27.1 | 4.66 |
| 3 | 10 % SiN₄ 30 minutes | 1.53 | 25.81 | 4.31 |
| 4 | 100 % SiN₄ 30 minutes | 1.98 | 25.83 | 7.3 |
| 5 | 10 % TEOS 1 minute | 5.3 | 35.4 | 4.82 |

As demonstrated herein, the amount of nitrogen in the capsules obtained after coating with tetra(n-propylamino)silane is higher compared to the alginate capsules not treated with the Si-N based coating solution, accordingly.

To conclude, the examples above demonstrate that it is possible to produce coated alginate capsules using Si-N-precursor. As demonstrated, the yeast cells contained in the alginate capsules present during coating demonstrates nearly 100 % recovery. Hence, the capsules according to the present invention represent a suitable system for immobilisation of bio molecules preserving its activity and functionality.

## Claims

1. A coated polymer system comprising silica-coated polymer and functional components **characterised in that** the coating of the polymer is a silica-coating whereby said silica-coating is obtained by using a Si-N precursor having at least two amino groups and whereby said coating is conducted in the presence of the functional components.

2. The coated polymer system according to claim 1, **characterised in that** i) the content of nitrogen in the coated polymer system is at least 5% higher than the content of nitrogen in the coated polymer system being coated with a Si-O precursor, and/or ii) the amount of nitrogen in the coated polymer system in the absence of a functional component is at least 0.1 wt-% of the weight of the total coated polymer system not containing functional component, preferably 0.5 wt.-%.

3. The coated polymer system according to claim 1 or 2, **characterised in that** the polymer is a biopolymer, in particular, a sol-gel biopolymer.

4. The coated polymer system according to claim 3, wherein the biopolymer is selected from polysaccharides, in particular, alginate, carrageenan, pectinate derivates, cellulose derivates, gums or from proteins.

5. The coated polymer system according to any one of the preceding claims **characterised in that** the functional component is a pH-sensitive and/or temperature-sensitive functional component.

6. The coated polymer system according to any one of the preceding claims, wherein the functional component is a biological component, in particular, cells, cellular components, and/or biologically active molecules, in particular, enzymes.

7. The coated polymer system according to any one of the preceding claims, wherein the functional component in the coated polymer system has a functionality of at least 50%, in particular, at least 70% compared to the activity of the functional component before incorporation into the coated polymer system.

8. The coated polymer system according to any one of the preceding claims, whereby said polymer is a sol-gel biopolymer containing said functional component and said polymer is coated with a silica based coating formed from Si-N precursors having at least two amino groups.

9. The coated polymer system according to any one of the preceding claims, whereby the functional component, in particular, the biologically active material, is present in the silica coating formed from the Si-N precursor having at least two amino groups.

10. A method for the preparation of a coated polymer system with functional components comprising the step of coating the polymer with Si-N precursor having at least two amino groups in the presence of said functional components.

11. The method according to claim 10, **characterised in that** the Si-N precursor is an aminosilane of the general formula (I) AₓSi(NR₁R₂)₄₋ₓ wherein A is independently selected from hydrogen, unsubstituted or substituted hydrocarbon, optionally containing heteroatoms, in particular, O, N, S, Si, Cl, Br, F and/or P; said hydrocarbon may be a saturated or unsaturated hydrocarbon in form of a linear, branched or cyclic hydrocarbon residue, furthermore, A may be an aromatic residue, preferably A is independently selected from optionally substituted C₁-C₂₀-alkyl-, C₆-C₂₄-aryl, in particular preferred a methyl, ethyl, propyl, vinyl, butyl or phenyl residue,
x is an integer of 0, 1 or 2
R₁ and R₂ are independently from each other hydrogen, unsubstituted or substituted hydrocarbon, optionally containing heteroatoms, in particular, O, N, S, Si, Cl, Br, F and/or P; said hydrocarbon may be a saturated or unsaturated hydrocarbon in form of a linear, branched or cyclic hydrocarbon residue, furthermore, R₁ and R₂ may be an aromatic residue, preferably R₁ and R₂ is independently selected from optionally substituted C₁-C₂₀-alkyl-, C₆-C₂₄-aryl, in particular preferred a methyl, ethyl, propyl, butyl, hexyl or phenyl residue.

12. The method according to claim 10, wherein the Si-N precursor having at least two amino groups is an oligo- or polysilazane.

13. The method according to any one of claims 10 to 12, **characterised in that** the polymer is a sol-gel biopolymer and the coating of the biopolymer is performed after formation of the sol-gel biopolymer whereby the functional component is present in the biopolymer and/or in the coating.

14. The method according to any one of claims 10 to 12, **characterised in that** the components forming the polymer, the Si-N precursor having at least two amino groups and the functional components are mixed with each other before forming the polymer and/or the coating.

15. The method according to any one of claims 10 to 14, whereby the coating is performed at a pH of 2 to 12, preferably 4 to 10, in particular at a pH of 6 to 8 and/or at a temperature of from -20°C to 80°C, in particular, 0°C to 60°C, preferably 20°C to 40°C.

16. The method according to any one of claims 10 to 15, **characterised in that** the functional component, in particular the biological active component, is present in the silica coating formed by the Si-N precursor having at least two amino groups.
